# EUROPEAN PATENT SPECIFICATION

(11) **EP 1 520 897 B1**
(45) Date of publication and mention of the grant of the patent: **30.07.2008**
(21) Application number: 04021750.7
(22) Date of filing: 14.09.2004
(51) Int. Cl.: C09J 7/04, C09J 7/02, A61F 5/443, A61F 13/02, A61L 15/22

(54) **Adhesive structures for attachment to skin**
Klebestruktur zur Befestigung auf der Haut
Structures adhésives pour attachement à la peau

(30) Priority: 18.09.2003 US 504291 P
(43) Date of publication of application: 06.04.2005
(73) Proprietor: Bristol-Myers Squibb Company, New York, N.Y. 10154 (US)
(72) Inventor: Fattman, George F., Mt. Laurel New Jersey 08054 (US)
(74) Representative: Holmes, Miles Keeton

(56) References cited:
- EP-A- 0 283 200
- DE-A- 19 524 661
- GB-A- 1 280 631
- US-A- 3 645 835
- US-A- 4 681 574
- US-A- 5 423 783
- US-A- 5 591 820
- US-A1- 2003 064 190
- JÜRGEN FALBE, MANFRED REGITZ: "Römpp Chemie Lexikon" 1991, GEORG THIEME VERLAG , STUTTGART, NEW YORK * page 3105 *

## Description

### Background

Adhesive structures for attachment to skin are typically composed of at least two materials including a pressure sensitive adhesive and a material adhered to that pressure sensitive adhesive on the opposite side from the surface where it would attach to the skin. That second material is often described as an adhesive backing or laminate. Many backings are possible but typically sheets of film, foam, or non-woven materials are used. The backing material may have many functions such as preventing the adhesive from adhering to a secondary surface, protecting the adhesive or the skin beneath the adhesive, or enabling attachment of a device to the skin through communication with the backing portion of the adhesive/backing structure.

Attachment of an adhesive structure to skin is unlike attachment to many other surfaces as a result of the fact that the skin gives off moisture. This process is called transepidermal water loss, or TEWL. A nominal value of TEWL for a healthy person at rest or who is not visibly perspiring is 250 grams of water per square meter of skin surface in 24 hours. It is well known that the presence of moisture at the interface between skin and an attaching structure typically causes a reduction in adhesion forces, unless the design or formulation of the attaching structure can account for the moisture from TEWL. A recent publication, Time-Dependent Changes in Dermal Peeling Force of Adhesive Tapes (Skin Research and Technology, 1999;5:33-36, Tokomura, et. al.) illustrates the rapid reduction in skin adhesion caused by the presence of moisture from TEWL.

Adhesive structures designed for attachment to skin can account for TEWL by one of two mechanisms, either absorption or transmission. An absorptive structure will capture TEWL moisture within itself, either within the adhesive or the backing or possibly both. To maintain adhesion, the rate of absorption should exceed the rate of TEWL moisture generation, and the absorbing capacity should be sufficient to accommodate moisture throughout the desired wearing time of the adhesive structure.

A transmitting adhesive structure will allow TEWL moisture to pass through the entire structure including both the adhesive and the backing. Unless the rate of transmission exceeds the rate of TEWL generation, the integrity of the bond between the adhesive structure and skin can be compromised.

One example of absorbent adhesive structures intended for attachment to skin are hydrocolloid adhesives, which are known for their ability to adhere securely for many days. Patents illustrative of this technology include US Patent No. 3,339,546 and US Patent No. 4,551,490. These adhesives comprise a hydrophobic, elastomeric, pressure sensitive adhesive matrix, into which dried powders of hydrophilic (absorbent) colloidal material are dispersed. Hydrocolloid adhesives manage perspiration by absorbing it. The absorbent powders transform the hydrophobic matrix into a hydrophilic formulation capable of absorbing as much as 6,000 grams/square meter/day of water.

When applied to skin, hydrocolloid adhesives absorb TEWL moisture and cause the structure to remain securely attached. When hydrated in this way, different hydrocolloid powders demonstrate varying degrees of their own adhesion to skin. Hydrocolloid adhesives adhere to skin even under conditions when the overall adhesive structure is occlusive, such as when the backing material chosen for these adhesives is itself impermeable to the passage of moisture or moisture vapor.

Examples of adhesive structures that transmit skin moisture are described in US Patent No. RE 31886 (Hodgson) and US Patent No. 4,681,574 (Eastman). Hodgson describes a moisture-vapor-permeable pressure sensitive adhesive for use on animal skin or nails comprising a pressure sensitive adhesive and a backing material, both of which are moisture vapor permeable. Eastman describes an ostomy appliance supported on the user's skin by a membrane adhesively held on the skin where the membrane and the adhesive layer are very thin, flexible, elastic and highly permeable to water vapor and oxygen transmission.

Both absorbent and transmitting types of adhesive structures for attachment to skin have limitations. Purely absorbent structures cannot absorb indefinitely before becoming saturated. Often they will become locally saturated in the area of skin attachment closest to the source of moisture. As they absorb their volume increases. The dimensions of the adhesive change, swelling in proportion to the amount absorbed. Upon saturation they can become non-adherent. A further drawback is that absorbent skin adhesive structures cannot differentiate between water-based fluids from different sources. For example, absorbent adhesives used in wound dressings will absorb moisture from TEWL as well as from wound effluent and other sources including those present when bathing, showering or swimming. As a result of this non-selective behavior, formulation of absorbent adhesives requires trade-offs in both absorption rate and capacity. They must absorb sufficient moisture at a high enough rate to be effective in managing TEWL. However, they must not absorb so much or so quickly that fluid from sources other than skin would quickly saturate the adhesive.

Despite these limitations, absorbent adhesives have been found to be especially useful in ostomy appliances. The most popular ostomy appliances comprise a skin barrier for protecting the peristomal area from stomal effluent, and a means for containing that effluent, such as a leak-proof, odor-proof pouch. Discs or wafers of absorbent adhesive have been found to be effective ostomy skin barriers. Not only do they manage perspiration, but also they remain securely adhered in the presence of stomal effluent. As previously mentioned, absorbent adhesives used as ostomy skin barriers will absorb water from any source present, including perspiration, stomal effluent, and from mucous from the stoma itself. Under normal conditions of wear, more effluent or mucous is present than perspiration, and the peristomal edge of the skin barrier is hydrated to a much greater extent than the adhesive at the skin interface.

Under certain circumstances the performance of an ostomy wafer comprised of an absorbent adhesive will be enhanced by absorption of effluent. If a small or moderate amount of moisture is absorbed, the increase in volume of the hydrated adhesive helps it to swell and seal the peristomal area. However, depending on the amount of moisture present, excess hydration at the edge of the skin barrier can lead to a loss of integrity in the adhesive to the point where it may lose adhesion. Further hydration can result in a loss of cohesion causing the adhesive to dissolve and possibly to expose peristomal skin. As a result, absorbent adhesives for ostomy skin barriers are formulated to balance their rate and capacity of absorption.

Different absorbent adhesive formulations for ostomy skin barriers may be balanced differently to favor certain conditions of wear. One formulation may be designed to enable the adhesive to remain adhered to skin in the presence of moderate to heavy perspiration, in cases where the amount of effluent present will not quickly lead to excessive hydration and loss of wafer integrity. Alternatively, another formulation may be designed to strongly resist stomal effluent, but at the risk of detachment during conditions of where perspiration is generated at a rate or in quantities exceeding the capability of the adhesive to absorb it.

Factors that affect the formulation balance are the cohesiveness of the pressure sensitive adhesive matrix and the absorbency of the individual absorbent particles added to the matrix. However, this balance is a general requirement for ostomy skin barriers that rely on absorption to remain attached to the skin. Inevitably, attack of the absorbing components by stomal effluent, or an overwhelming of the absorbing characteristics of the barrier by perspiration remain significant failure modes for these devices.

Another characteristic of absorbent adhesives is that the strength of the bond to skin depends on the amount of perspiration absorbed at the adhesive-skin interface. As more perspiration is absorbed the strength of that bond eventually decreases. Depending on the wearing time of the adhesive and the amount of perspiration present, this decline can mean a less secure attachment as the wearing time progresses. It also means that the wearer cannot depend on a consistent strength of attachment. Again, if the amount of perspiration generated approaches the limit of rate or capacity of the adhesive to absorb it, the security of the bond may become inadequate. For an ostomy device, this condition would be unacceptable if it occurred during the wearing time of the device. For that reason absorbent adhesives for ostomy skin barriers are typically formulated to favor absorption at the risk of being more susceptible to attack by stomal effluent.

A further limitation of absorbent adhesives is their thickness and flexibility. To adequately incorporate absorbent materials into their formulation, the thickness of these adhesives is typically 0.76mm (0.030 inches) or more. Rheological changes in the elastomeric matrix brought about by the incorporation of high concentration of absorbent fillers causes absorbent adhesives to be rather stiff and somewhat inflexible during wear.

Transmitting adhesive structures (also known as breathable, or moisture vapor permeable structures) for attachment to skin also have limitations. First, both the adhesive and the backing must be permeable to moisture vapor. Only a limited number of materials can meet this requirement. Commercially, these special materials are more costly to produce and tend to be tailored to specific, relatively smaller volume applications, as compared with olefin-based plastics, which have a wider range of applications and are commodity materials. As a result, the components of breathable adhesive structures are typically more expensive and less available compared with the components of non-permeable pressure sensitive adhesives or backing films.

When these structures are used to attach devices to the skin the composition of moisture vapor permeable adhesive structures, especially permeable backings, causes them to be chemically (thermodynamically), thermally, and/or mechanically incompatible with many other materials. If the adhesive and backing material are incompatible, anchorage of the adhesive can be less than sufficient to keep these components together during use. Anchorage is also especially important during removal of the device since the backing greatly facilitates removal of the adhesive from skin. Compatibility between the adhesive and the backing must be sufficient to withstand stresses between these materials during both use and removal of the structure from skin.

Compatibility between the backing and any device component that may require attachment to the backing is also essential. Most breathable backing films are typically composed of specialty polymer resins like poly(ether-amide), poly(esteramide), poly(ether-ester), and poly(urethane). These polymers have no or very limited compatibility with the commodity grade, olefin-based plastics like poly (ethylene), ethylene copolymers, poly (propylene), poly (styrene), etc, which are commonly used to manufacture disposable medical devices. Additionally, the hard segments of these block copolymers have relatively high glass transition temperatures. As a result, processes commonly used to assemble medical devices to adhesive structures like heat welding, sonic welding, radio frequency welding, etc. are not effective for attaching olefin-based device components to films composed of these specialty polymers. In order to use these processes to attach a device to a breathable backing material, the composition of that device must be limited to more expensive, less available, breathable polymer resins like those listed above. Less efficient processes using additional materials could be employed to assemble the device to the adhesive structure, for example by gluing, but again the compatibility requirements for the various components must be satisfied. It is believed that the quality, security and reliability of the gluing process are inferior compared with processes for thermal welding components together.

In order to obtain a satisfactory MVTR, breathable adhesive structures are typically produced at a thickness of 0.1mm (0.004 inch) or less. At this dimension, the structure is difficult to apply to skin in one smooth application. In most cases the flimsiness of the structure causes it to drape upon itself or to wrinkle before it can be applied to the skin surface. Application of a medical device of this construction by a person with limited dexterity and/or some amount of vision impairment can be difficult to the extent that the performance of the device can be compromised. A removable paper substrate supplied on the uncoated side of the backing film of the final adhesive structure may provide rigidity to the structure during application to skin, but then this paper must be removed without interfering with the bonded adhesive structure. This removal requires additional work and, again, good dexterity and visual acuity.

Breathable adhesive structures would be challenged by several specific manifestations of the difficulties enumerated above if they would be employed as ostomy skin barriers. First, acceptable ostomy appliances require containment of effluent, typically in an odor-proof pouch. To achieve the odor barrier properties necessary for an effective ostomy pouch, barrier films are used that are not compatible with the high MVTR materials that typically comprise the backings of breathable adhesive structures. As a result the two dissimilar materials are not easily joined together. Selection of alternative materials for either component would be complicated by the fact that achieving odor-proof and breathable characteristics in the same material may be inherently inconsistent. One additional limitation of breathable structures for ostomy skin barriers is the fact that at least some amount of fluid must be taken up by the structure in order to enable it to be transmitted. As a result some of the same limits of absorbent structures would also be factors in the performance of breathable structures used as ostomy skin barriers, to the extent that they also are absorbent. In particular it has been found that a breathable adhesive structure used as an ostomy skin barrier was readily attacked by stoma effluent through breathable backing because it provided an ineffective barrier to that material. Once exposed to the effluent the breathable adhesive became ineffective for adhering to or protecting the peristomal area.

From the perspective of cost and compatibility, a backing composed of olefin polymers would be preferred. However, adhesion to the skin would be limited as olefin based plastic sheets are essentially moisture vapor impermeable. Several techniques exist by which these sheets can be perforated to allow the ready passage of moisture vapor from one side to the other. US Patent Nos. 3,881,489 (Hartwell), 4,472,328 (Sugimoto, et al), 4,317,792 (Raley, et al), and 4,777,073 (Sheth) illustrate various methods by which olefin films may be perforated to yield a breathable sheet from impervious materials. Unfortunately, these perforated films also suffer from a number of drawbacks.

First, when perforated backing materials are coated with a suitable breathable adhesive, the MVTR of the entire structure has been found to be less than that of the individual components. The magnitude of this decrease is determined by the ratio of open area contributed by the perforations to the entire area of the sheet. This reduction occurs because the surface area of breathable adhesive that is available for the passage of moisture is reduced to that of the open area created by the perforations, and a small, occluded region around those openings from which the adhesive can still transmit moisture out of the structure.

The experimental data in the following table illustrates the magnitude of MVTR reduction that was realized in structures of this kind. Structure 2 suggests that the MVTR of the polyurethane adhesive is approximately 1,500 gsm/24 hrs (grams per square meter per 24 hours), and that the MVTR for the perforated EVA foam is 1,300 gsm/24 hrs. When these two materials were laminated together the MVTR of the composite structure was substantially reduced below those of the materials measured separately.

**Table 1: Moisture Vapor Transmission Rate of Various Materials and Structures by ASTM E96**

| Experiment | Backing Material | Adhesive | MVTR (grams/square meter/24 hrs) |
|---|---|---|---|
| 1 | Polyurethane Film - Continuous Sheet | None | 3,000 |
| 2 | Polyurethane Film - Continuous Sheet | Polyurethane | 1,500 |
| 3 | EVA Foam - Continuous Sheet | None | < 25 |
| 4 | EVA Foam - Perforated Sheet (Open Area = 1.8 %) | None | 1,300 |
| 5 | EVA Foam - Perforated Sheet (Open Area = 3 %) | Polyurethane | 226 |

| | | | |
|---|---|---|---|
| Materials: EVA Foam: Voltek 6EO 0.0031 poly(ethylene vinyl acetate) cross linked foam Polyurethane Adhesive: Refer to US Patent No. 5,591,820 (Kydonieus) and International Application No. WO 99/38900 (Hostettler & Wachtel) Polyurethane Film: Rexam Custom Ltd. polyurethane film, PUR-9801 | | | |

Perforating olefinic films to render them moisture vapor permeable also suffers from the limitation that the mechanical properties of the sheet are reduced. As a result the strength of the structure and the durability of the bond between the sheet and any device attached to it will be reduced.

In many applications, at least one component of a breathable adhesive structure, usually the backing film, should be to some extent resistant to the passage of liquid water. One reason for this requirement is the need to protect the adhesive and whatever it covers from external fluids or potential fluid-bome substances. For example, in an ostomy pouch, fecal effluent emanating from a stoma must be kept away from the skin. In this case the adhesive structure functions as a skin barrier, protecting the peristomal skin from excoriation. In that case the adhesive structure must be liquid impervious. Stomal effluent has been found to be an aggressive surface-active agent, enabling it to penetrate very small holes and into interfaces of imperfectly bonded materials. Another example is the need for wound dressings to protect wounds from viral or bacterial contamination. This protection could not be adequately accomplished if the dressing permits liquid water to pass through it.

A final limitation on breathable adhesive structures for attachment to skin is that the components must be compatible with skin, thus limiting the number of the available materials even further.

An improvement over the prior art would result from an adhesive structure for attachment to skin comprised of inexpensive and readily available materials, that would enable assembly of devices by the most efficient methods, and would be free from the various limitations of absorbent or breathable adhesive structures. Objects of this invention include adhesive structures for attachment to the skin that address these prior art limitations, adhesive structures comprising olefin based backing materials that transmit moisture, and ostomy wafers comprising them. It is also an object of this invention to improve the performance of ostomy skin barriers by designing the adhesive wafer to effectively manage perspiration while resisting attack by stomal effluent.

### Description of the Invention

The present invention in defined in the claims. In development of the invention, pressure sensitive adhesives comprised of polyurethane formulations of the kind described in US Patents Nos. 5,591,820 (Kydonieus) and 6,359,100 (Hostettler & Wachtel) were coated onto silicone release paper and laminated with various backings chosen to obtain a range of moisture vapor transmission rates (MVTR). The compositions of these structures are shown in Table 2. The experimental structures show an MVTR above TEWL (Structure C), approximately at TEWL (Structure D) and below TEWL (Structure E). Structure B has a backing film that is essentially impermeable to moisture vapor, so the structure has a low MVTR. Structure C has a backing film that has a high MVTR, so the MVTR essentially reflects the MVTR of the adhesive alone. Structure D was produced by laminating the above polyurethane adhesive to a composite backing material, the backing material being similar to those described by US Patents Nos. 4,995,930 (Merz et al.) or 5,733,628 (Pelkie), in which a non-woven material is bonded to a perforated film. The adhesive of Structure D was laminated with the perforated film side of the composite backing material, the film first being corona treated to improve anchorage of the adhesive. Structures A and E are commercially available adhesive structures for attachment to skin and are not described by the two patents referenced immediately above.

**Table 2: MVTR of Materials and Structures by ASTM E96**

| Structure Identification | Adhesive | Backing Material | Backing Format | MVTR (grams/square meter/24 hrs) |
|---|---|---|---|---|
| A | Acrylic | Polyester | Non-Woven | 1,900 |
| B | Polyurethane | Polyethylene Backing | Embossed Continuous Film | <40 |
| C | Polyurethane | Polyurethane Film | Continuous Film | 1,500 |
| D | Polyurethane | Polyethylene and | Non-Woven | 120 |
| | | EVA | Perforated Film | |
| E | Polyurethane | Commercial PUR Wound Dressing | Polyurethane Foam | 2,000 |

| | | | | |
|---|---|---|---|---|
| Materials: Acrylic adhesive: poly( 2-ethyl hexylacrylate) Polyurethane Adhesive: Refer to US Patent Nos. 5,591,820 (Kydonieus) and 6,359,100 (Hostettler & Wachtel) Polyurethane Film: Rexam Custom Ltd. polyurethane film, PUR-9801 EVA perforated film: 40 mesh perforated film of poly(ethylene vinylacetate), 2 mils thick. | | | | |

As noted above, these structures exhibited a wide range of MVTR and were suitable to test the impact of this parameter on adhesion to skin. These structures were prepared into sheets from which were cut 25 mm by 76 mm (1 inch by 3 inch) strips. The strips were applied to the backs of healthy volunteers. After time intervals of 1, 4, and 7 days, the adhesive strips were visually observed and removed under well controlled conditions. A visual rating of the skin adhesion of each strip was made. The results for skin adhesion are summarized in Table 3.

**Table 3: Surface Adhesion (Scale: 0=Dreasing off, 7=All corners adhering firmly)**

| Time Interval Number of Observations | Mean Adhesion Scores | | | | | Between-Treatment Friedman p-value | Fishers LSD Comparisons² |
|---|---|---|---|---|---|---|---|
| | A | B | C | D | E | | |
| Day 1 N | 6.72 25 | 6.76 25 | 6.48 25 | 6.60 25 | 6.52 25 | 0.1137 | Not Applicable |
| Day 4 N | 6.08 25 | 5.72 25 | 5.12 25 | 6.04 25 | 4.96 25 | 0.2955 | Not Applicable |
| Day 7 N | 2.40 25 | 1.64 25 | 2.92 25 | 3.72 25 | 1.88 25 | <0.0001¹ | D vs. CABE C vs. BE |

| | | | | | | | |
|---|---|---|---|---|---|---|---|
| ¹Significant differences among products. ² Products listed first within each comparison exhibited better adhesion. | | | | | | | |

For the structures listed in Table 2, any build up of moisture between the skin and adhesive was expected to reduce their adhesion. It has surprisingly been discovered that a permeable adhesive can remain reliably attached to skin when paired with an olefinic backing material that does not allow the passage of moisture vapor at a rate exceeding average TEWL of human skin. The D adhesive structure, with relatively low MVTR, was nevertheless the best performing structure on Day 7, and performed equivalently to the more permeable structures on Days 1 and 4.

This discovery lead to additional experiments wherein polyurethane adhesive was laminated with various backing materials to form structures with the MVTRs shown in Table 4.

**Table 4: MVTR of Additional Materials and Structures by ASTM E96**

| Structure Identification | Adhesive | Backing Material | Backing Format | MVTR (grams/square meter/24 hrs) |
|---|---|---|---|---|
| F | Polyurethane | Polyester | 48 grams/meter² Non-Woven | 1,550 |
| G | Polyurethane | Polyethylene | 80 grams/meter² Non-Woven | 1,680 |
| H | Polyurethane (Laminated with perforated film) | Polyethylene and | 100 grams/meter² Non-Woven | 355 |
| | | EVA Film | 40 Mesh Perforated, 2 mil Film | |
| I | Polyurethane (Laminated with perforated film) | Polyethylene | 80 grams/meter² Non-Woven | 715 |
| | | EVA | 40 Mesh Perforated, 2 mil Film | |
| J | Polyurethane (Laminated with Non-woven) | EVA Film | 40 Mesh Perforated, 2 mil Film | 1,692 |
| | | Polyethylene | 80 grams/meter² Non-Woven | |
| K | Silicone Adhesive - Dow Bio-PSA 7-4501 (Laminated with Non-woven) | EVA Film | 40 Mesh Perforated, 2 mil Film | 1,760 |
| | | Polyethylene | 80 grams/meter² Non-Woven | |
| L | Silicone Adhesive - Dow Bio-PSA 7-4501 (Laminated with Non-woven) | EVA Film | 40 Mesh Perforated, 2 mil Film | 1,683 |
| | | Polyethylene | 80 grams/meter² Non-Woven | |
| M | Hydrogel (Laminated with Non-woven) | EVA Film | 40 Mesh Perforated, 2 mil Film | 140 |
| | | Polyethylene | 80 grams/meter² Non-Woven | |
| N | Hydrogel (Laminated with Non-woven) | EVA Film | 40 Mesh Perforated, 2 mil Film | 490 |
| | | polyethylene | 80 grams/meter² Non-Woven | |

The difference between structure "I" and structure "J" is that the adhesive was laminated with the non-woven side of the composite structure with the unexpected result that the MVTR increased dramatically. Structure J provides a breathable adhesive with an olefinic backing film, the structure having a high MVTR and also being protected from penetration by liquid water across the majority of its surface, thus addressing many limitations of adhesive structures known in the prior art. It was also found by experiment that the bond to the adhesive is greatly improved when the strength of the non-woven fiber is increased (for example a polyamide fiber), or when the non-woven fabric has a networked structure, for example from point welding or orientation (for example, stretching or combing). Similar backing films, including microporous films of the kind described by Sheth in US Patent No. 4,777,073 may also be combined with a suitable non-woven material to obtain increased MVTR compared with the microporous film as the sole adhesive backing. In this case additional protection of the structure is obtained since films of this kind are reported to be liquid impermeable.

Adhesive sheets with structures similar to those described above and comprising adhesives laminated with olefinic based films, each component having an MVTR above the TEWL from a healthy normal human at rest, can also be converted into ostomy skin barriers. The backing films can, for example, be microporous films, perforated films, non-wovens and combinations thereof and similar backing materials including at least one olefin based polymer. The adhesives can, for example, be any adhesives suitable for adhering to the skin with a MVTR greater than approximately 250 gsm/day. For example, adhesives can be based on polyurethanes, polyacrylates, polysiloxanes, their copolymers and hydrogels. Pouches can be attached to the adhesive structure and evaluated for their ability to resist intestinal fluids.

Further, olefinic films known to be impermeable to both ileo fluid and moisture vapor can be attached to the portion of the adhesive structure that would be exposed to ileo fluid as a result of that surface being located inside the attachment perimeter of the pouch. The portion of the structure residing outside the pouch then would include only the adhesive and the backing film, both of which transmit moisture above the nominal TEWL of a healthy human at rest. This can also reduce the zone of attack of the adhesive structure by the ileo fluid.

## Claims

1. An adhesive structure for attachment to skin comprising an adhesive having a skin attachment surface for attachment to the skin, the adhesive transmitting moisture vapor at a rate exceeding 250 grams per square meter per 24 hours according to ASTM E96, and a backing material on the opposite side of the adhesive to the skin attachment surface, the backing material comprising a non-woven fabric and a perforated or microporous film, the backing material including an olefin based polymer and also transmitting moisture vapor at a rate exceeding 250 grams per square meter per 24 hours according to ASTM E96, and wherein the adhesive comprises polyurethane, polyacrylate, polysiloxane, their copolymers, or hydrogel adhesives.

2. The adhesive structure of claim 1 wherein the non-woven fabric or the perforated or microporous film comprises polyethylene, polypropylene, polyether, polyester, polyamide, or their copolymers.

3. The adhesive structure of claim 1 wherein the microporous film does not pass liquid water within about 6 hours.

4. The adhesive structure of claim 1 for use with an ostomy appliance.

5. An ostomy appliance comprising an adhesive structure as defined in any preceding claim.

6. The ostomy appliance of claim 5 wherein the portion of the backing material enclosed or covered by attachment of the effluent containing portion of the appliance is itself covered with a film that is impermeable to stomal effluent.

7. The ostomy appliance of claim 6 wherein the surface of the adhesive structure is impermeable to stomal effluent within the portion of the surface of the backing material enclosed or covered by attachment of the effluent containing portion of the appliance, said adhesive structure having an MVTR exceeding 250 grams per square meter per 24 hours at some other surface portion of the adhesive structure.

8. The ostomy appliance of claim 7 wherein the adhesive structure is created from an impermeable olefin-based-polymer containing film that is then perforated or otherwise made moisture vapor permeable.

9. The adhesive structure of any preceding claim wherein the non-woven fabric is processed to increase its strength.

## Patentansprüche

1. Eine Klebestruktur zur Befestigung auf der Haut, enthaltend einen Klebstoff mit einer Hautbefestigungsfläche zur Befestigung auf der Haut, wobei der Klebstoff Wasserdampf mit einer Rate durchlässt, die 250 g pro m² pro 24 h gemäß ASTM E96 übersteigt, und ein Trägermaterial auf der zu der Hautbefestigungsfläche entgegengesetzten Seite des Klebstoffs, wobei das Trägermaterial einen Vliesstoff und einen perforierten oder mikroporösen Film enthält, das Trägermaterial ein Olefin-basiertes Polymer beinhaltet und Wasserdampf auch mit einer Rate durchlässt, die 250 g pro m² pro 24 h gemäß ASTM E96 übersteigt, und wobei der Klebstoff Polyurethan, Polyacrylat, Polysiloxan, deren Copolymere oder Hydrogel-Klebstoffe enthält.

2. Klebestruktur nach Anspruch 1, wobei der Vliesstoff oder der perforierte oder mikroporöse Film Polyethylen, Polypropylen, Polyether, Polyester, Polyamid oder deren Copolymere enthält.

3. Klebestruktur nach Anspruch 1, wobei der mikroporöse Film kein flüssiges Wasser innerhalb von ungefähr 6 Stunden durchlässt.

4. Klebestruktur nach Anspruch 1 zur Verwendung mit einer künstlichen Einrichtung eines Darmausgangs.

5. Künstliche Einrichtung eines Darmausgangs, enthaltend eine Klebestruktur nach einem der vorangehenden Ansprüche.

6. Künstliche Einrichtung eines Darmausgangs nach Anspruch 5, wobei der Teil des Trägermaterials, der durch die Befestigung des dem Ausfluss enthaltenden Teils der Einrichtung umgeben oder bedeckt ist, selbst mit einem Film bedeckt ist, der undurchlässig für Stomaausflüsse ist.

7. Künstliche Einrichtung eines Darmausgangs nach Anspruch 6, wobei die Fläche der Klebestruktur undurchlässig für Stomaausfüsse innerhalb des Teils der Fläche des Trägermaterials ist, der durch die Befestigung des den Ausfluss enthaltenden Teils der Einrichtung umgeben oder bedeckt ist, die Klebestruktur einen MTVR hat, der an einigen anderen Flächenteilen der Klebestruktur 250 g pro m² pro 24 h übersteigt.

8. Künstliche Einrichtung eines Darmausgangs nach Anspruch 7, wobei die Klebestruktur aus einem undurchlässigen Olefin-basierten Polymer enthaltenden Film erstellt ist, der dann perforiert oder anderweitig wasserdampfdurchlässig gemacht wird.

9. Klebestruktur nach einem der vorangehenden Ansprüche, wobei der Vliesstoff zum Erhöhen seiner Festigkeit bearbeitet ist.

## Revendications

1. Structure adhésive pour adhésion à la peau comprenant un adhésif ayant une surface d'adhésion à la peau pour une adhésion à la peau, l'adhésif ayant un taux de perméabilité à l'humidité dépassant 250 grammes par mètre carré par 24 heures en accord avec la norme E96 de l'ASTM, et un matériau de support sur le côté opposé de l'adhésif à la surface d'adhésion à la peau, le matériau de support comprenant un tissu non tissé et un film perforé ou microporeux, le matériau de support comprenant un polymère de structure oléfine ayant aussi un taux de perméabilité à l'humidité supérieur à 250 grammes par mètre carré par 24 heures en accord avec la norme E96 de l'ASTM, et dans laquelle l'adhésif comprend le polyuréthane, le polyacrylate, le polysiloxane, leurs copolymères, ou adhésifs hydrogels.

2. Structure adhésive selon la revendication 1 dans laquelle le tissu non tissé ou le film perforé ou microporeux comprend le polyéthylène, le polypropylène, le polyéther, le polyester, le polyamide, ou leurs copolymères.

3. Structure adhésive selon la revendication 1 dans laquelle le film microporeux ne laisse pas passer l'eau liquide pendant 6 heures environ.

4. Structure adhésive selon la revendication 1 pour une utilisation avec un appareillage stomique.

5. Appareillage stomique comprenant une structure adhésive telle que définie dans l'une quelconque des revendications précédentes.

6. Appareillage stomique selon la revendication 5 dans lequel la partie de matière absorbante enfermée ou couverte par adhésion de la partie contenant l'effluent de l'appareillage est elle-même couverte avec un film qui est imperméable à l'effluent gastro-duodénal.

7. Appareillage stomique selon la revendication 6 dans lequel la surface de la structure adhésive est imperméable à l'effluent gastro-duodénal dans la partie de la surface du matériau absorbant enfermée ou couverte par l'adhésion de la partie de l'appareillage contenant l'effluent, ladite structure adhésive ayant un MVTR supérieur à 250 grammes par mètre carré par 24 heures à une autre partie de surface de la structure adhésive.

8. Appareillage stomique selon la revendication 7 dans lequel la structure adhésive est créée à partir d'un film contenant un polymère de structure oléfine qui est alors perforé ou sinon fabriqué de façon à être perméable à l'humidité.

9. Structure adhésive selon l'une quelconque des revendications précédentes dans laquelle le tissu non tissé est traité pour augmenter sa résistance.
